# EUROPEAN PATENT SPECIFICATION

(11) **EP 3 594 683 B1**
(45) Date of publication and mention of the grant of the patent: **16.08.2023**
(21) Application number: 18719637.3
(22) Date of filing: 08.03.2018
(51) Int. Cl.: A61K 31/517, G01N 33/574, G01N 33/92, A61P 35/04, A61P 35/00

(54) **MEDICAL USES OF APOLIPOPROTEIN A AND ACTIVATORS THEREOF**
MEDIZINISCHE VERWENDUNGEN EINES APOLIPOPROTEINS A UND AKTIVATOREN DAVON
USAGES MÉDICAUX DE L'APOLIPOPROTÉINE A ET D'ACTIVATEURS DE CELLE-CI

(30) Priority: 08.03.2017 ES 201730304
(43) Date of publication of application: 15.01.2020
(73) Proprietor: Fundación Imdea Alimentación, 28049 Madrid (ES)
(72) Inventor: AGUIRRE PORTOLÉS, Cristina, 28049 Madrid (ES); REGLERO RADA, Guillermo, 28049 Madrid (ES); RAMÍREZ DE MOLINA, Ana, 28049 Madrid (ES)
(74) Representative: Hoffmann Eitle
(86) International application number: PCT/IB2018/000317
(87) International publication number: WO 2018/162984

(56) References cited:
- WO-A2-2015/173633
- US-A1- 2013 281 397
- CHUN YEH ET AL: "Pravastatin inhibits tumor growth through elevating the levels of apolipoprotein A1", ADVANCES IN DIGESTIVE MEDICINE, vol. 3, no. 1, 1 March 2016 (2016-03-01), pages 3-10, XP055485001, ISSN: 2351-9797, DOI: 10.1016/j.aidm.2015.03.003
- GILHAM DEAN ET AL: "RVX-208, a BET-inhibitor for treating atherosclerotic cardiovascular disease, raises ApoA-I/HDL and represses pathways that contribute to cardiovascular disease", ATHEROSCLEROSIS, ELSEVIER, AMSTERDAM, NL, vol. 247, 22 January 2016 (2016-01-22), pages 48-57, XP029468988, ISSN: 0021-9150, DOI: 10.1016/J.ATHEROSCLEROSIS.2016.01.036
- AGUIRRE-PORTOLÉS CRISTINA ET AL: "ABCA1 overexpression worsens colorectal cancer prognosis by facilitating tumour growth and caveolin1-dependent invasiveness, and these effects can be ameliorated using the BET inhibitor apabetalone", MOLECULAR ONCOLOGY, vol. 12, no. 10, 17 September 2018 (2018-09-17), pages 1735-1752, XP055778041, ISSN: 1574-7891, DOI: 10.1002/1878-0261.12367 Retrieved from the Internet: URL:https://onlinelibrary.wiley.com/doi/fu ll-xml/10.1002/1878-0261.12367>

## Description

### TECHNICAL FIELD OF THE INVENTION

The present invention relates to an apolipoprotein A activator for use in the treatment or prevention of cancer according to the appended claims.

### BACKGROUND OF THE INVENTION

The latest World Cancer Report showed that about 14 million new cases were recorded in 2012 and there were 8.2 million deaths associated with this disease (International Agency for Research on Cancer, 2014). According to predictions, the number of new cases will increase by about 70% in the next 20 years. Most of the steps leading to the formation of a tumor have been studied in detail to date; however, it can be considered that there is still limited knowledge about metastatic processes. Primary tumors cause 10% of cancer deaths, whereas metastasis is responsible for patient death in 90% of the cases. Taking these premises into account, the need to find new mechanisms which allow stopping the colonization of distant tissues far from the primary tumor is evident.

In 2014, Teodoro Vargas *et al.* demonstrated that the activation of ABCA1, ACSL1, AGPAT1, and SCD genes was the main factor in the malignant progression in stage II colorectal cancer (II-CRC) patients (T Vargas, J Moreno-Rubio, J Herranz, et al. Genes associated with metabolic syndrome predict disease-free survival in stage II colorectal cancer patients: A novel link between metabolic dysregulation and colorectal cancer. Mol Oncol. December 2014; 8 (8):1469-81). Among them, ABCA1 (ATP-binding cassette transporter A1) is a transporter protein the main function of which is to remove from the cell cholesterol that will be taken up by apolipoprotein A-1 (ApoA1) to give rise to high-density lipoproteins (HDLs). In 2013, Mohelnikova-Duchonova *et al.* reported increases in the expression levels of ABCA1 in one of the most therapy-resistant cancers, i.e., pancreatic cancer (Mohelnikova-Duchonova B BV, Oliverius M, Honsova E, et al. Differences in transcript levels of ABC transporters between pancreatic adenocarcinoma and nonneoplastic tissues. Pancreas 2013; 42:707-716). However, Angela H. Ting's laboratory shows decreased ABCA1 levels in malignant prostate cancer patients as compared to those patients with benign tumors (Lee BH, Taylor MG, Robinet P, et al. Dysregulation of cholesterol homeostasis in human prostate cancer through loss of ABCA1. Cancer Res 2013; 73:1211-8). In the case of ovarian cancer, increased ABCA1 levels have been linked to a poor prognosis of the disease (Hedditch EL, Gao B, Russell AJ, et al. ABCA transporter gene expression and poor outcome in epithelial ovarian cancer. J. Natl. Cancer Inst. 2014; 106(7):766-76.); however, the decrease in expression levels by means of hypermethylation of the ABCA1 promoter has also been linked to a poor prognosis (Chou JL, Huang RL, Shay J, et al. Hypermethylation of the TGF-β target, ABCA1 is associated with poor prognosis in ovarian cancer patients. Clin Epigenetics, 14 January 2015; 7(1):1). Chun Yeh et al., Advances in Digestive Medicine, vol. 3, no. 1, p. 3-10, 2016 disclose decreased apolipoprotein A1 levels in colorectal cancer cells and elevation of apolipoprotein A1 to inhibit tumor proliferation.

There is therefore a need in the art to identify new treatments for cancer patients, as well as methods for identifying patients who may have a good response to said treatment.

### BRIEF DESCRIPTION OF THE INVENTION

The authors of the present invention have surprisingly observed that apolipoprotein A, a mimetic peptide thereof, or an activator thereof are capable of inhibiting the proliferation, migration, and invasion of cancer cells, particularly cancer cells having an overexpression of ABCA1. The inventors have also discovered that patients diagnosed with tumors in advanced stages of the disease have decreased ApoA1 expression levels in a tumor sample with respect to patients diagnosed with tumors in earlier stages. The inventors have also discovered that patients suffering from metastasis have decreased plasma ApoA1 levels with respect to patients diagnosed with a primary tumor.

Therefore, the invention relates to a composition comprising an apolipoprotein A activator with the following structure: 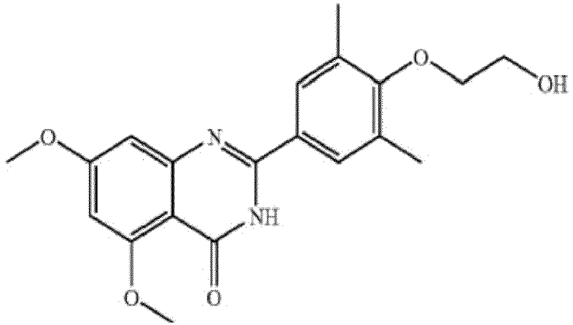 for use in a method of inhibiting the proliferation, migration, and invasion of cancer cells, in a cancer patient with decreased ApoA1 plasma levels with respect to a reference value, wherein reference value for the ApoA1 level is the mean plasma ApoA1 level of a group of subjects not suffering from cancer, and wherein said cancer cells are characterized by having an overexpression of ABCA1.

In a preferred embodiment, the cancer cells are colorectal cancer cells.

In a preferred embodiment, the apolipoprotein A activator forms a lipoprotein complex comprising a fraction comprising said apolipoprotein A activator and a lipid fraction. In a preferred embodiment, the lipid fraction of the lipoprotein complex consists essentially of sphingomyelin and about 3% by weight of a negatively charged phospholipid, and the molar ratio of the lipid fraction with respect to the apolipoprotein A activator fraction is in the range of about 200:1 to 2:1.

### DESCRIPTION OF THE DRAWINGS

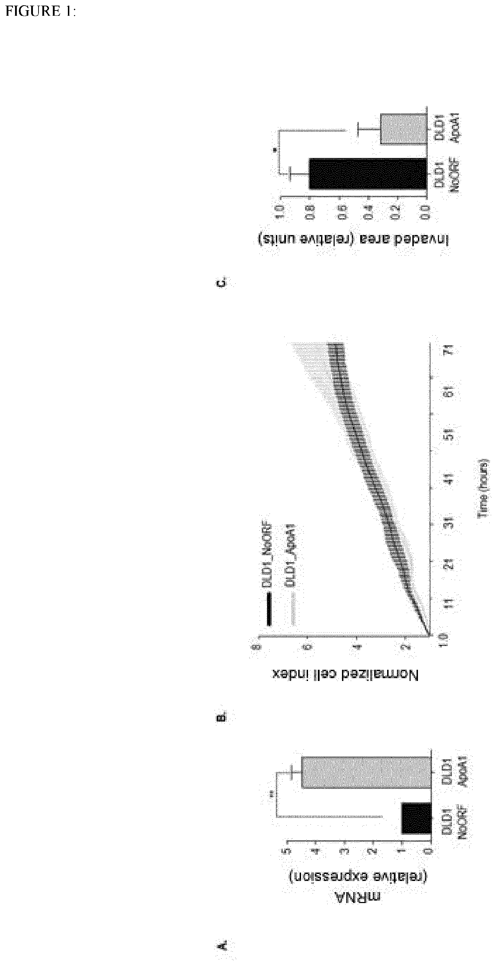

**FIGURE 1. A)** ApoA1 messenger RNA expression levels analyzed by means of quantitative PCR (**p-value = 0.0020). **B)** Proliferation curve in a viability assay carried out by means of *XCELLigence* technology (ACEA Biosciences, Inc.). The graph shows the growth of control DLD1 cells versus DLD1 cells (colon cancer-derived epithelial cells) overexpressing ApoA1. **C)** Invasion assays carried out in Corning^{®} BioCoat^{™} Matrigel plates (#354480). Each point in the graph represents an analyzed insert. The results are shown normalized with respect to the control and represent the invaded area in the lower region of the matrix through which the cells migrate (*p-value = 0.0218).

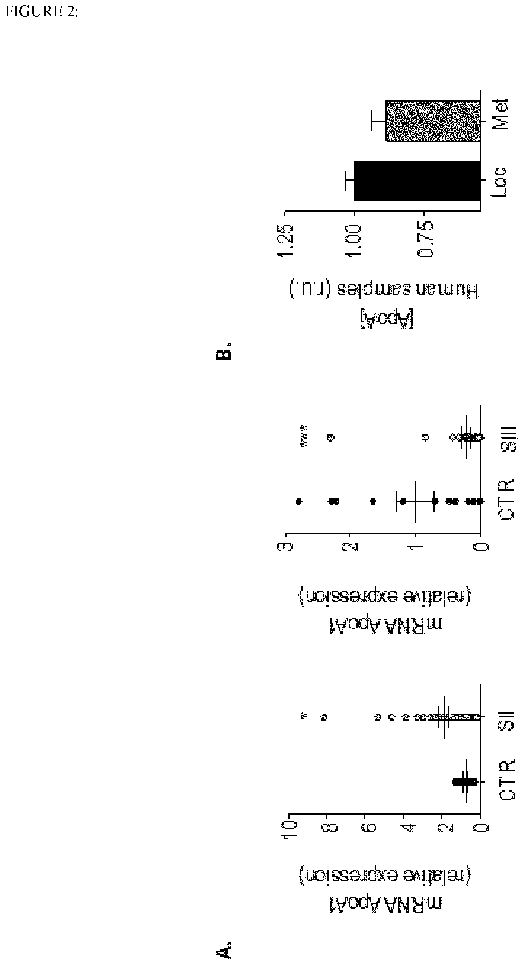

**FIGURE 2. A)** ApoA1 mRNA levels in patients from Hospital Universitario de la Paz. The CTR samples correspond to healthy tissue from a specific patient. Each SII or Sill sample corresponds to a tumor sample from the same patient whose healthy tissue was also analyzed (**p-value = 0.0141; ***p-value = 0.0005). **B)** ApoA1 levels analyzed by means of an ELISA assay in plasmas derived from patients from Hospital Universitario de la Paz diagnosed with a primary colon tumor versus metastatic patients.

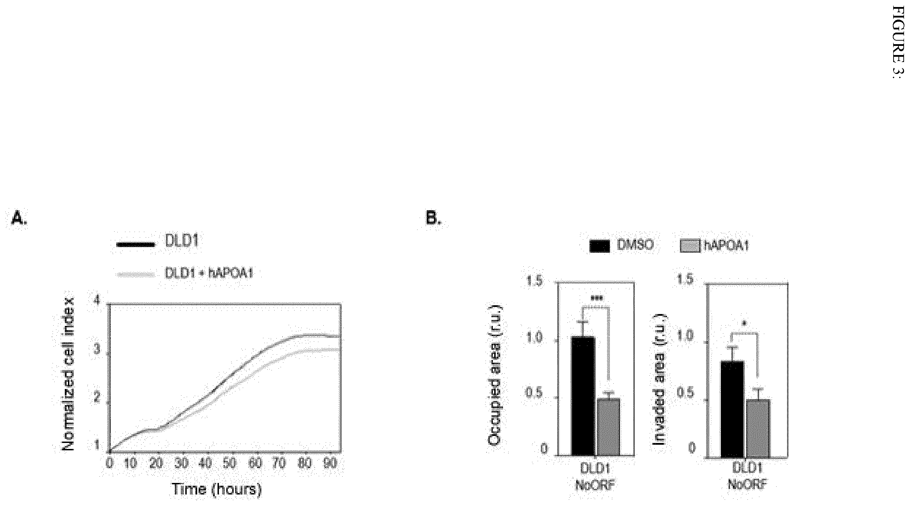

**FIGURE 3. A)** Proliferation curve in a viability assay carried out by means of *XCELLigence* technology (ACEA Biosciences, Inc.). The graph shows the growth of control DLD1 cells versus DLD1 cells (colon cancer-derived epithelial cells) in the absence and presence of ApoA1 (40 µg/ml for 96 hours before the experiment and 20 µg/ml during data acquisition). **B)** Migration and invasion assays carried out in Coming^{®} BioCoat^{™} Control plates (#354578) and Corning^{®} BioCoat^{™} Matrigel plates (#354480), respectively. Each point in the graph represents an analyzed insert. The assays were carried out in the presence of 40 µg/ml of ApoA 172 hours before the experiment. The assays were performed in the presence of 20 µg/ml of ApoA1.
   The results are shown normalized with respect to the control and represent the occupied or invaded area in the lower region of the matrix through which the cells migrate. (****p-value = 0.0001; *p-value = 0.0386).

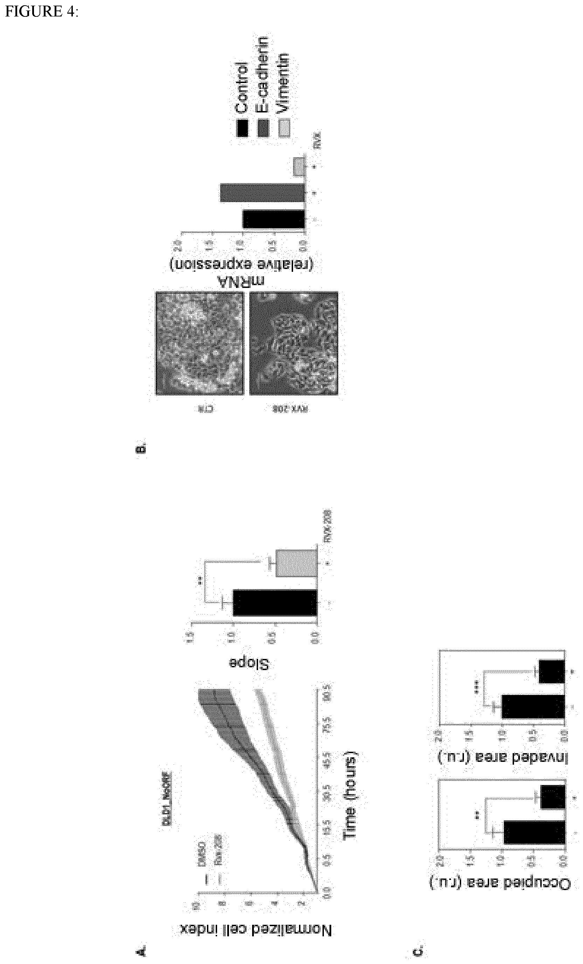

**FIGURE 4. A)** Proliferation curve in a viability assay carried out by means of *XCELLigence* technology. Growth of DLD1 cells treated with DMSO as the drug carrier and with Rvx-208 (30 µM). The significant differences existing in the slope of the graph, and therefore in the growth dynamics of the treated cells are shown in the right panel (**p-value = 0.0087). **B)** Image representing the untreated cell cultures and those treated with Rvx-208. Expression levels of proteins E-cadherin and vimentin in the presence and absence of Rvx-208. Analysis performed by means of quantitative PCR. The relative expression levels normalized with respect to the untreated control are shown. **C)** Migration and invasion assays in the presence of Rcx-208.

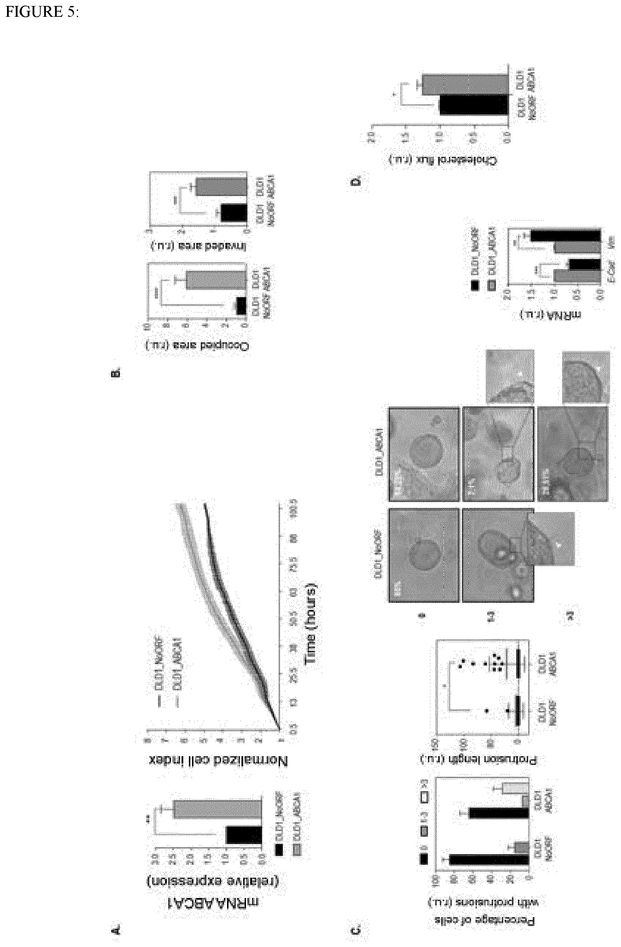

**FIGURE 5. A)** ABCA1 messenger RNA levels in the DLD1 cell line generated for the stable overexpression thereof (* *p-value = 0.093). Proliferation curve in a viability assay carried out by means of *XCELLigence* technology. The normalized cell index for the control cell line (NoORF, No Open Reading Frame) and the line overexpressing ABCA1 is shown. **B)** Migration and invasion assays. The data was collected and processed 72 hours after starting the experiment. (****p-value < 0.0001; ***p-value = 0.0009). **C)** Matrigel-embedded spheroid invasion assay. The percentage of spheroids having protrusions invading the matrix (representative images in the central panels), as well as the maximum length thereof in the different cell cultures, are shown. E-Cadherin and vimentin messenger RNA levels are shown. **D)** Cholesterol flux in control cells and cells overexpressing ABCA1).

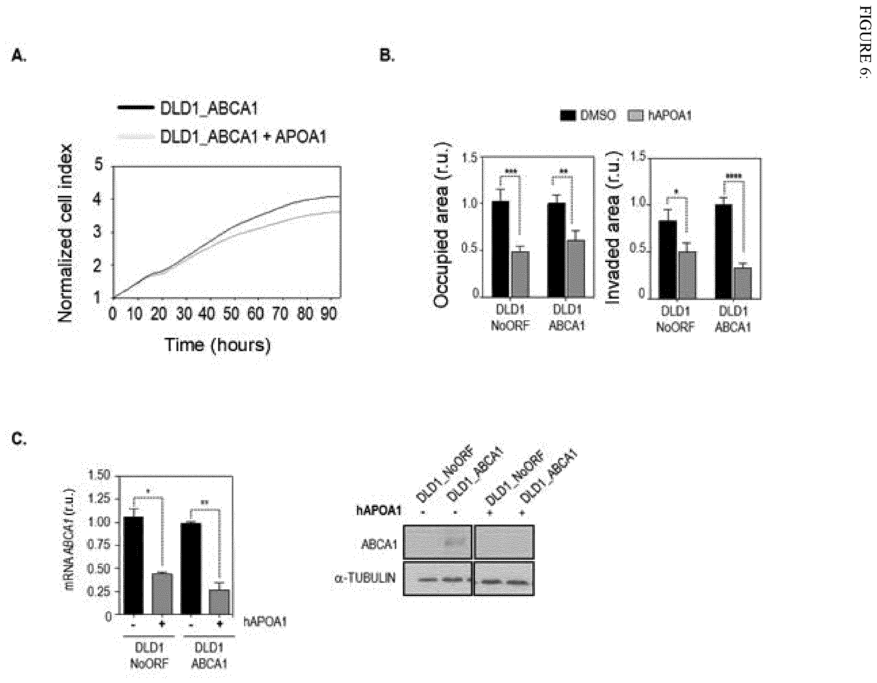

**FIGURE 6. A)** Proliferation curve in a viability assay carried out by means of *XCELLigence* technology. The results for the stable cell line overexpressing ABCA1 in the absence and presence of ApoA1 are shown. The cells were treated for 72 hours before the experiment with 40 µg/ml of ApoA1 and with a concentration of 20 µg/ml during acquisition. **B)** Migration and invasion assays. The method followed was the same as the one described in Figure 1. (**p-value = 0.003; ****p-value < 0.0001). C) ABCA1 messenger RNA level (left panel) and ABCA1 protein level (right panel) in the presence and absence of treatment with ApoA1. Results are shown both for the control cells and for those cells overexpressing the transporter.

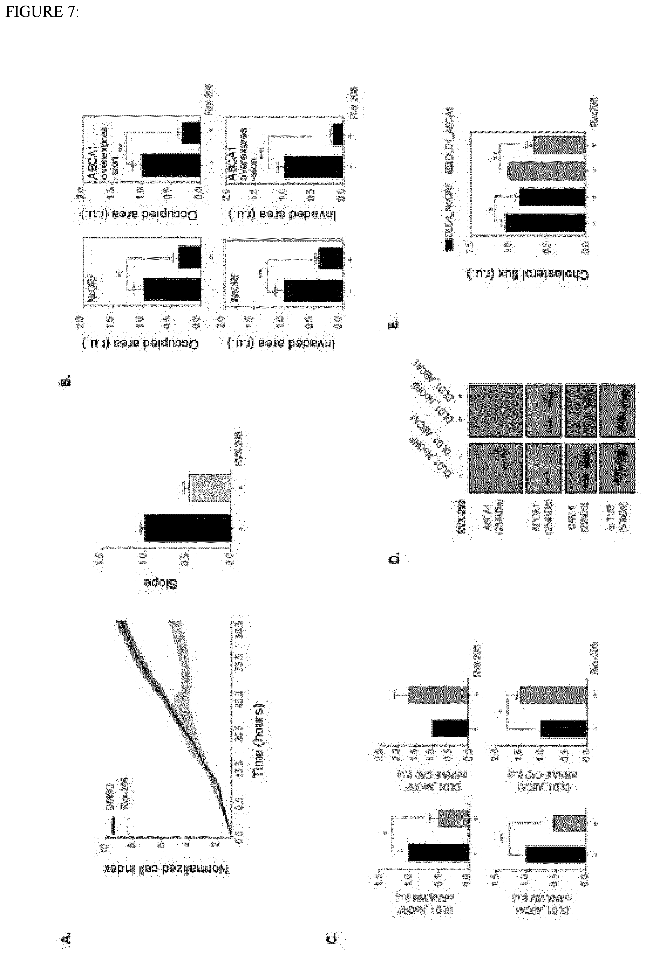

**FIGURE 7. A)** Proliferation curve in a viability assay carried out by means of *XCELLigence* technology. Growth of DLD1 cells treated with DMSO as the drug carrier and with Rvx-208 (30 µM). The significant differences existing in the slope of the graph, and therefore in the growth dynamics of the treated cells are shown in the right panel (****p-value < 0.0001). **B)** Migration and invasion assays. The method followed was the same as the one described in Figure 2 (*p-value = 0.0407; ****p-value <0.0001). **C)** Expression levels of proteins E-cadherin and Vimentin in the presence and absence of Rvx-208. Analysis performed by means of quantitative PCR. The relative expression levels normalized with respect to the untreated control are shown. D) ABCA1, ApoA1, caveolin-1, and α-tubulin immunodetection by means of Western blot. α-tubulin acts as load control. The results of the cells treated with Rvx-208 (30 µM) or with the DMSO carrier are shown. E) Cholesterol flux in cells treated with Rvx-208.

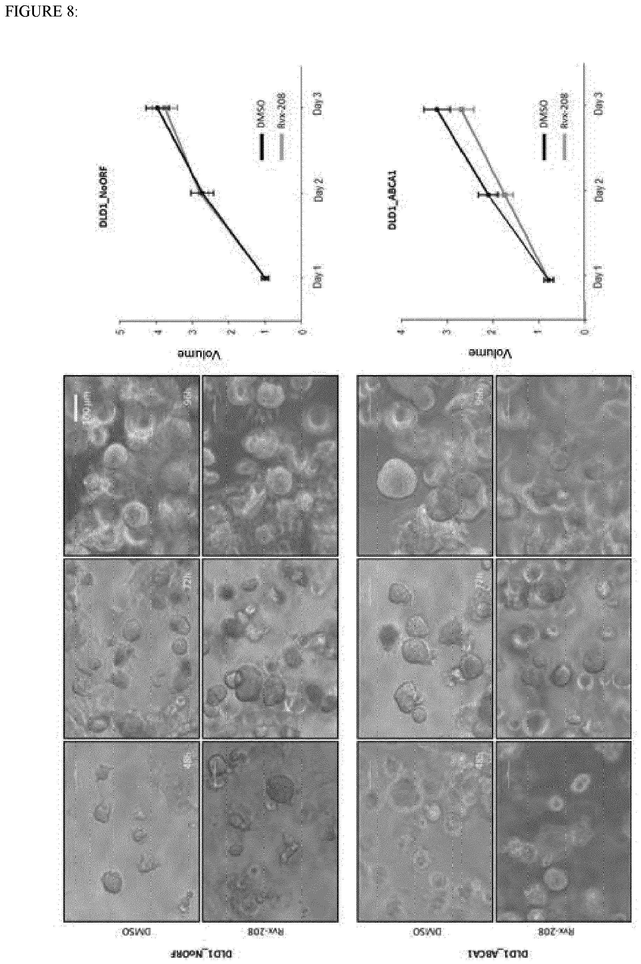

**FIGURE 8:** A) Growth of spheroids derived from control cells and cells overexpressing ABCA1. The top panels correspond to the control treatment (carrier), the bottom panels correspond to treatment with Rvx-208. The growth curves represent the mean volume of a total of 30 spheroids per cell line.

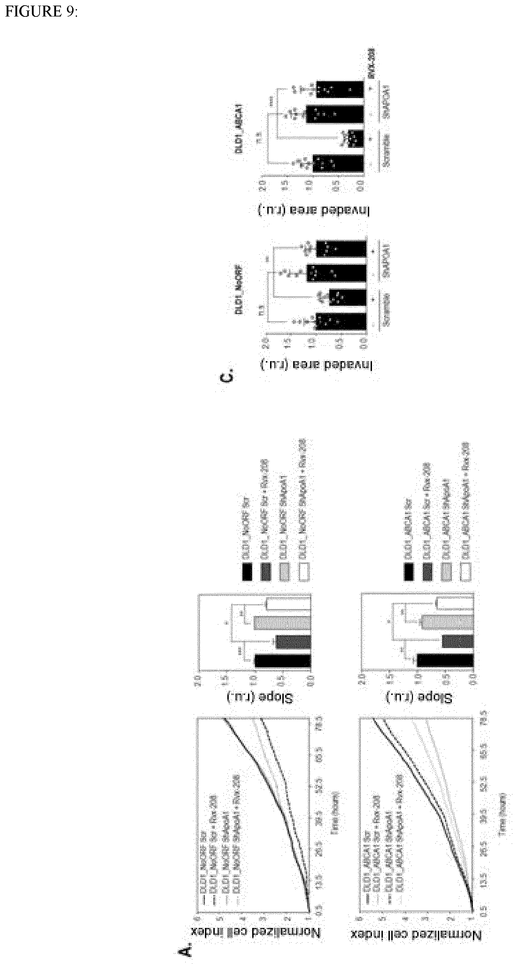

**FIGURE 9: A)** Proliferation curve in a viability assay carried out by means of *XCELLigence* technology. Growth of DLD1 cells treated with DMSO as the drug carrier and with Rvx-208 (30 µM). Control cells (scramble, SCR) are shown in black. Cells transfected with anti-ApoA1 short hairpin are shown in gray. The significant differences existing in the values of the slope of the graphs on the left-hand side, and therefore in the growth dynamics of the treated cells, are shown in the right panels (^{∗}p-value = 0.0471; ^{∗∗}p-value = 0.0067; ^{∗∗∗∗}p-value <0.0001). B) Invasion assays in cells with silenced ApoA1 expression.

### DETAILED DESCRIPTION OF THE INVENTION

### Medical use of apolipoprotein A, a mimetic peptide thereof, or an activator thereof

The inventors have discovered that apolipoprotein A, a mimetic peptide thereof, or an activator thereof are capable of inhibiting the proliferation, migration, and invasion of cancer cells, particularly cancer cells having an overexpression of ABCA1.

Therefore, the invention relates to the apolipoprotein A activator apabetalone (RVX-208), having the following structure: 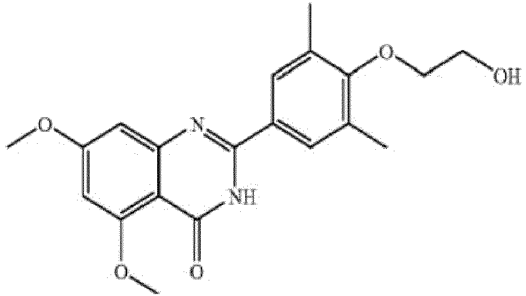 for use in a method of inhibiting the proliferation, migration, and invasion of cancer cells, in a cancer patient with decreased ApoA1 plasma levels with respect to a reference value, wherein reference value for the ApoA1 level is the mean plasma ApoA1 level of a group of subjects not suffering from cancer, and wherein said cancer cells are characterized by having an overexpression of ABCA1

In the context of the present invention, apolipoprotein A is selected from ApoA1, ApoA2, ApoA4, and ApoA5. In a particular embodiment, apolipoprotein A is ApoA 1.

In the context of the present invention, "apolipoprotein" is understood to be a protein containing and transporting lipids in blood. An apolipoprotein is an amphipathic heteroprotein with a lipid prosthetic group that is part of the lipoproteins. The prefix apo- of the term apolipoprotein means that it is the fundamental protein part of lipoproteins, but it should not be confused with the apoprotein thereof, which is the protein part. In any case, the term apolipoprotein used in the context of the present invention interchangeably refers to the apolipoprotein and the corresponding apoprotein.

In the context of the present invention, "ApoA1" is understood to be a gene encoding apolipoprotein A1. The human gene is shown in the Ensembl database with accession number ENSG00000118137.

In the context of the present invention, "ApoA2" is understood to be a gene encoding apolipoprotein A2. The human gene is shown in the Ensembl database with accession number ENSG00000158874.

In the context of the present invention, "ApoA4" is understood to be a gene encoding apolipoprotein A4. The human gene is shown in the Ensembl database with accession number ENSG00000110244.

In the context of the present invention, "ApoA5," also known as APOA-V, RAP3, APOAV, is understood to be a gene encoding apolipoprotein A5. The human gene is shown in the Ensembl database with accession number ENSG00000110243.

In the context of the present invention, the terms "cancer" and "tumor" refer to the physiological condition in mammals characterized by uncontrolled cell growth in which the equilibrium between cell multiplication and cell death is disrupted. The cancer to be treated in the context of the present invention can be any type of cancer or tumor. These tumors or cancer include, and are not limited to, hematologic cancers (for example, leukemias or lymphomas), neurological tumors (for example, astrocytomas or glioblastomas), melanoma, breast cancer, lung cancer, head and neck cancer, gastrointestinal tumors (for example, stomach cancer, pancreatic cancer, or colon cancer), liver cancer (for example, hepatocellular carcinoma), renal cell cancer, genitourinary tumors (for example, ovarian cancer, vaginal cancer, cervical cancer, bladder cancer, testicle cancer, prostate cancer), bone tumors, and vascular tumors.

In a preferred disclosure, the cancer is a primary tumor. In another preferred disclosure, the cancer is a metastasis. In a preferred embodiment, the cancer is a colorectal cancer.

As it is used herein, the term "colorectal cancer" (CRC) includes any type of neoplasias of the colon, rectum, and appendix and refers to early and late adenomas, carcinoma, as well as hereditary, familial, or sporadic cancer. In the staging systems for colorectal cancer classification, the colon and rectum are treated as a single organ. The invention contemplates treatment of the different stages of colorectal cancer such as stages A, B, C1, C2, and D according to the Dukes classification, stages A, B 1, B2, B3, C1, C2, C3, and D according to the Astler-Coller classification, stages T1, T2, T3, N0, N1, N2, M0, and M1 according to the TNM system, as well as stages 0, I, II, III, and IV according to the AJCC (American Joint Committee on Cancer) classification. According to the tumor/node/metastasis (TNM) staging system of the American Joint Committee on Cancer (AJCC) (Greene et al. (eds.), cancer of the AJCC Staging Manual. 6th edition. New York, NY: Springer, 2002), the different stages of colorectal cancer are defined as follows:
- Tumor: T1: the tumor invades the submucosa, T2: the tumor invades the muscularis propria, T3: the tumor invades the muscularis propria in the subserosa, or the pericolic or perirectal tissues; T4: the tumor directly invades other organs or structures, and/or penetrates.
- Node: N0: no metastasis to regional lymph nodes; N1: metastasis in 1 to 3 regional lymph nodes; N2: metastasis in 4 or more regional lymph nodes.
- Metastasis: M0: no distant metastasis present; M1: distant metastasis present.

Therefore, stages 0, I, II, III, and IV would have the following features:
- S-0: Tis/N0/M0 (Tis=carcinoma in situ);
- S-I: T1/N0/M0 or T2/N0/M0;
- S-II: T3/N0/M0 or T4/N0/M0;
- S-III: Any T/N1/M0 or any T/N2/M0
- S-IV: Any T/any N/M1

In the context of the invention, "treatment of cancer" is understood as the administration of the apolipoprotein A activator according to the invention to prevent or delay the onset of symptoms, complications, or biochemical indications of the cancer or tumor, to alleviate its symptoms, or to stop or inhibit its development and progression such as, for example, the onset of metastasis. The treatment can be a prophylactic treatment for delaying the onset of the disease or for preventing the manifestation of its clinical or subclinical symptoms, or a therapeutic treatment for eliminating or alleviating symptoms after the manifestation of the disease, or in relation to the surgical or radiotherapy treatment thereof. The administration of the apolipoprotein A activator according to the invention can be done simultaneously or consecutively with any other treatment already known in the treatment of cancer.

The cancer is characterized by being found in a patient having a high ABCA1 gene expression level with respect to a reference value, by having a high level of a metabolite resulting from ABCA1 activity with respect to a reference value, and/or by having a high level of a parameter associated with ABCA1 activity.

In the context of the present invention, "ABCA1," also known as CERP, HDLDT1, ABC1, ABC-1, TGD, is understood to be a gene encoding the cholesterol transporter "ATP binding cassette subfamily A member 1." The human gene is shown in the Ensembl database with accession number ENSG00000165029. ABCA1 gene expression levels can be quantified based on the RNA resulting from the transcription of said gene (mRNA), or alternatively based on the complementary DNA (cDNA) of said gene. Therefore, in a particular embodiment of the invention, the quantification of ABCA1 gene expression levels comprises quantifying the messenger RNA of the ABCA1 gene, a fragment of said mRNA, complementary DNA of the TFF 3 gene, a fragment of said cDNA, or the mixtures thereof. Additionally, the method of the invention may include performing an extraction step for the purpose of obtaining total RNA, which extraction step can be performed by means of conventional techniques (Chomczynski et al, Anal. Biochem., 1987, 162:156; Chomczynski P., Biotechniques, 1993, 15:532).

Virtually any conventional method can be used within the scope of the invention for detecting and quantifying the levels of the mRNAs encoded by the ABCA1 gene or of their corresponding cDNA. In a non-limiting and illustrative manner, the levels of the mRNAs encoded by said gene can be quantified by means of using conventional methods, for example, methods which comprise amplifying the mRNA and quantifying the amplification product of said mRNA, such as electrophoresis and staining, or alternatively by means of Southern blot and using suitable probes, Northern blot and using probes specific for the mRNA of the gene of interest (ABCA1) or of its corresponding cDNA, S1 nuclease mapping, RT-CSF, hybridization, microarrays, etc., preferably by means of real-time quantitative PCR using a suitable marker. Similarly, the levels of the cDNA corresponding to said mRNA encoded by the ABCA1 gene can also be quantified by means of using conventional techniques; in this case, the method of the invention includes a synthesis step for synthesizing the corresponding cDNA by means of reverse transcription (RT) of the corresponding mRNA followed by amplification and quantification of the amplification product of said cDNA. Conventional methods for quantifying expression levels can be found, for example, in Sambrook, 2001. "Molecular cloning: a Laboratory Manual," 3rd edition, Cold Spring Harbor Laboratory Press, New York, Vol. 1-3. In a particular embodiment, ABCA1 gene expression levels are quantified by means of a quantitative polymerase chain reaction (PCR).

On the other hand, for putting the invention into practice, besides quantifying ABCA1 gene expression levels, the expression levels of the protein encoded by said gene, i.e., the ABCA1 protein, or any functionally equivalent variant of said ABCA1 protein, can also be quantified. Therefore, in a particular embodiment, the quantification of the levels of the protein encoded by the ABCA1 gene comprises quantifying the ABCA1 protein.

The ABCA1 protein expression level can be quantified by means of any conventional method which allows detecting and quantifying said protein in a sample from a subject. In a non-limiting and illustrative manner, the levels of said protein can be quantified, for example, by means of using antibodies capable of binding to ABCA1 (or to fragments thereof containing an antigenic determinant), and the subsequent quantification of the complexes that are formed. The antibodies used in these assays may or may not be labeled. Illustrative examples of markers that can be used include radioactive isotopes, enzymes, fluorophores, chemiluminescent reagents, enzyme substrates or cofactors, enzyme inhibitors, particles, dyes, etc. There is a wide range of known assays that can be used in the present invention, using non-labeled antibodies (primary antibody) and labeled antibodies (secondary antibody); these techniques include, among others, Western blot, ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemical techniques, techniques based on the use of biochips or protein microarrays including specific antibodies, or assays based on colloidal precipitation in formats such as dipsticks. Other ways of detecting and quantifying said ABCA1 protein include affinity chromatography techniques, ligand binding assays, etc. Commercial anti-ABCA1 antibodies which can be used in the context of the present invention are available on the market. In a particular embodiment, the levels of the protein encoded by the ABCA1 gene are quantified by means of Western blot, immunohistochemical technique, or ELISA.

In a preferred embodiment, the ABCA1 expression level in the primary tumor is at least twice the reference value.

As it is used herein in the context of the invention, the term "reference value" refers to a laboratory value used as a reference for values/data obtained by means of laboratory examinations of subjects or samples collected from the subjects. The reference value or reference level can be an absolute value, a relative value, a value having an upper and/or lower limit, a range of values, an average value, a median value, an average value, or a value in comparison with a particular control value or baseline value. A reference value can be based on a value of an individual sample, such as, for example, a value obtained for a sample from the subject who is being evaluated, but at a time before developing the disease, or a sample originating from a non-cancerous tissue. The reference value may be based on a large number of samples, for example, a population of subjects of the coinciding chronological age group, or may be based on a group of samples which includes or excludes the sample to be tested. Several considerations are taken into account when determining the reference value. Such considerations include, among others, the age, weight, sex, general physical condition of the patient and the like. For example, a group having the same numbers of at least 2, at least 10, at least 100, and preferably more than 1000 subjects, preferably classified according to the preceding considerations, for example according to various age categories, is taken as a reference group.

The reference value for the ABCA1 expression level is preferably the mean ABCA1 expression level in a group of non-tumor tissue samples. In a preferred embodiment, the non-tumor tissue is from a healthy subject who does not suffer from cancer. In an additional preferred embodiment, the non-tumor tissue is from a subject suffering from a cancer different from the patient. In another preferred embodiment, the non-tumor tissue is from the actual patient.

In another embodiment, the amount of the marker in a sample from a subject can be determined directly in relation to the reference value (for example, in terms of an increase or a decrease, or an increase in the number of folds or a decrease in the number of folds). Advantageously, this may allow comparing the amount of the marker in the sample from the subject with the reference value (i.e., measuring the relative amount of the marker in the sample from the subject with respect to the subject with the reference value) without having to first determine the respective absolute amounts of the marker. Once this reference value has been established, the level of this marker expressed in the tumor tissues of the subjects can be compared with this reference value, and an "increased" or "decreased" level is therefore assigned to it. For example, an increase in expression levels above the reference value of at least 1.1-fold, 1.5-fold, 2-fold, 5-fold, 10-fold, 20-fold, 30-fold, 40-fold, 50-fold, 60-fold, 70-fold, 80-fold, 90-fold, 100-fold, or even higher in comparison with the reference value is considered an "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold, or even lower in comparison with the reference value is considered a "decreased" expression level.

As it is used herein, the term "patient" or "subject" refers to all animals classified as mammals and includes, but is not limited to, pets and farm animals, primates, and humans, for example, human beings, non-human primates, cows, horses, pigs, sheep, goats, dogs, cats, or rodents. The subject is preferably a male or female human of any age or race.

The metabolite resulting from ABCA1 activity can be selected from phosphatidylcholine, phosphatidylserine, sphingomyelin, cholesterol, cholesterol esters, and HDL. In a preferred embodiment of the invention, the metabolite resulting from ABCA1 activity is cholesterol.

The metabolite resulting from ABCA1 activity, preferably cholesterol, can be determined in a biofluid sample by any means known to one skilled in the art.

In the present invention, the term "sample" or "biological sample" refers to biological material isolated from a subject. The biological sample may contain any biological material suitable for detecting the desired marker and may comprise cells and /or non-cellular material from the subject. The sample can be isolated from any suitable biological fluid or tissue, such as blood, blood plasma, serum, urine, cerebrospinal fluid (CSF), or feces, for example. The samples may be biofluid samples. The terms "biological fluid" and "biofluid" are used interchangeably herein and refer to aqueous fluids of biological origin. The biofluid can be obtained from any location (such as blood, plasma, serum, urine, bile, cerebrospinal fluid, vitreous or aqueous humor, or any bodily secretion), an exudate (such as the liquid obtained from an abscess or any other infection or inflammation site), or the liquid obtained from a joint (for example, a normal joint or a joint affected by a disease such as rheumatoid arthritis).

In a preferred embodiment, the biofluid is a blood sample from the patient.

The reference value for a metabolite resulting from ABCA1 activity is preferably the mean concentration level of said metabolite resulting from ABCA1 activity in a group of biofluid samples in subjects not suffering from the disease.

The parameter associated with ABCA1 activity can be selected from the half-life of caveolin-1 (CAV1), the plasma HDL level, and the intracellular cholesterol ester level. In a preferred embodiment of the invention, the parameter associated with ABCA1 activity is the half-life of caveolin-1 (CAV1). In the context of the present invention, "CAV1," also known as BSCL3, CAV, PPH3, LCCNS, CGL3, VIP21, MSTP085, is understood to be a gene encoding caveolin 1. The human gene is shown in the Ensembl database with accession number ENSG00000105974. In the context of the invention, the term "half-life of caveolin-1" refers to the time required for 50% of a specific amount of caveolin-1 to degrade. Her *et al.* (Nam-Hu Her, et al., Cell Cycle (2013), 12:10, 1521-1535) demonstrate in a prostate cancer cell line that ABCA1 depletion results in a drastic decrease of the CAV1 protein due to the decreased stability thereof, whereas the overexpression of ABCA1 results in an increased stability of CAV1 due to an increase in membrane cholesterol levels.

In preferred embodiments of the invention, the reference value is the ABCA1 expression level in a non-tumor tissue, the level of the metabolite resulting from ABCA1 activity in a healthy subject who does not suffer from cancer, or the value of the half-life of caveolin-1 in a subject who does not suffer from cancer. In the context of the present disclosure, "apolipoprotein A activator" is understood to be any molecule capable of increasing apolipoprotein A messenger RNA expression, as well as increasing apolipoprotein A concentration, giving rise to an increase in high-density lipoprotein (HDL).

The apolipoprotein A activator according to the invention is apabetalone (RVX-208). RVX-208 (2-(4-(2-hydroxyethoxy)-3,5-dimethylphenyl)-5,7-dimethoxyquinazolin-4(3H)-one) is described in detail in Figure 20 of patent document US 8,053,440. Therefore, the apolipoprotein A activator has the following structure:

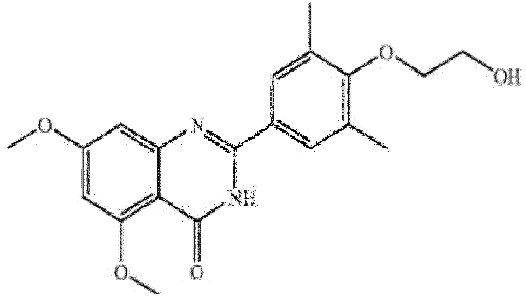

In the context of a non-claimed aspect, the term "mimetic peptide" refers to a small chain similar to the protein designed for imitating a peptide. They are typically derived from modifying an existing peptide, or by means of designing similar peptide-imitating systems, such as peptoids and β-peptides. Regardless of the approach, the altered chemical structure is design for advantageously adjusting molecular properties such as biological activity or stability. This may play a role in the development of drug-like compounds from existing peptides. These modifications involve changes in the peptide that will not occur naturally (such as an altered linear structure and the incorporation of non-natural amino acids).

In a preferred aspect, the mimetic peptide of ApoA1 is a lipoprotein complex comprising an apolipoprotein ApoA-1 fraction and a lipid fraction.

In the context of the present invention, lipoproteins are macromolecular complexes made up of proteins and lipids. Lipoproteins transport fats throughout the entire body. They are spherical, water-soluble, formed by (a) a non-polar lipid core (esterified cholesterol and triglycerides) and (b) a polar outer layer formed by apoproteins, phospholipids, and free cholesterol. Lipoproteins are classified into different groups according to their density, the higher the density, the higher the protein content (the larger the diameter, the higher the lipid content): chylomicrons, very low-density lipoproteins (VLDLs), intermediate-density lipoproteins (IDLs), low-density lipoproteins (LDLs), and high-density lipoproteins (HDLs). Each type of lipoprotein has a composition and a proportion characteristic of apolipoproteins (ApoA, ApoB, ApoC, ApoE).

The lipid fraction of the lipoprotein complex comprises phosphatidylcholine (lecithin), phosphatidylethanolamine (cephalin), phosphatidylserine, phosphatidylinositol, phosphatidylglycerol, sphingomyelin, ganglioside, cerebroside, or combinations thereof.

The molar ratio of the lipid fraction with respect to the apolipoprotein ApoA-1 fraction may be comprised in the range of about 200:1 to 2:1; about 180:1 to 2:1; about 160:1 to 2:1; about 140:1 to 2:1; about 120:1 to 2:1; about 100:1 to 2:1; about 90:1 to 2:1; about 80:1 to 2:1; about 70:1 to 2:1; about 60:1 to 2:1; about 50:1 to 2:1; about 40:1 to 2:1; about 30:1 to 2:1; about 20:1 to 2:1; about 10:1 to 2:1; about 5:1 to 2:1.

In a preferred embodiment, the lipid fraction of the lipoprotein complex consists essentially of sphingomyelin and about 3% by weight of a negatively charged phospholipid, and the molar ratio of the lipid fraction with respect to the apolipoprotein ApoA-1 fraction is in the range of about 200:1 to 2:1.

In non-claimed aspect, the mimetic peptide of ApoA1 is CER-001: (www.cerenis.com/en/our-therapies/cer-001).

CER-001 comprises ApoA1, sphingomyelin and DPPG in a phospholipid:lipoprotein weight ratio of 1:2.7; and with a sphingomyelin:DPPG weight ratio of 97:3. CER-001 is prepared according to the method described in Example 4 of patent document WO 2012/109162.

### Method for determining the stage of a colorectal tumor in a patient (not according to the invention)

The inventors have discovered that patients diagnosed with tumors in advanced stages of the disease have decreased ApoA1 expression levels in a tumor sample with respect to patients diagnosed with tumors in earlier stages. Likewise, the inventors have discovered that patients suffering from metastasis have decreased plasma ApoA1 levels with respect to patients diagnosed with a primary tumor.

Therefore, a non-claimed aspect relates to a method for determining the stage of a colorectal tumor in a patient, where the method comprises determining ApoA1 levels in a biofluid from said patient, wherein decreased ApoA1 levels with respect to a reference value indicate a more advanced stage of the tumor.

The terms "cancer," "colorectal cancer," and "patient" have been described in detail above and are similarly applicable to the methods according to the method of this aspect.

In the present invention, the term "sample" or "biological sample" refers to the biological material isolated from a subject. The biological sample may contain any biological material suitable for detecting the desired marker and may comprise cells and /or non-cellular material from the subject. The sample can be isolated from any suitable biological fluid or tissue, such as blood, blood plasma, serum, urine, cerebrospinal fluid (CSF), or feces, for example. The samples may be biofluid samples. The terms "biological fluid" and "biofluid" are used interchangeably herein and refer to aqueous fluids of biological origin. The biofluid can be obtained from any location (such as blood, plasma, serum, urine, bile, cerebrospinal fluid, vitreous or aqueous humor, or any bodily secretion), an exudate (such as the liquid obtained from an abscess or any other infection or inflammation site), or the liquid obtained from a joint (for example, a normal joint or a joint affected by a disease such as rheumatoid arthritis).

In a preferred disclosure, the biofluid is a plasma sample from the patient.

The ApoA1 protein expression level can be quantified by means of any conventional method which allows detecting and quantifying said protein in a biofluid sample from a subject. In a non-limiting and illustrative manner, the levels of said protein can be quantified, for example, by means of using antibodies capable of binding to ApoA1 (or to fragments thereof containing an antigenic determinant) and the subsequent quantification of the complexes that are formed. The antibodies used in these assays may or may not be labeled. Illustrative examples of markers that can be used include radioactive isotopes, enzymes, fluorophores, chemiluminescent reagents, enzyme substrates or cofactors, enzyme inhibitors, particles, dyes, etc. There is a wide range of known assays that can be used in the present invention, using non-labeled antibodies (primary antibody) and labeled antibodies (secondary antibody); these techniques include, among others, Western blot, ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemical techniques, techniques based on the use of biochips or protein microarrays including specific antibodies, or assays based on colloidal precipitation in formats such as dipsticks. Other ways of detecting and quantifying said ApoA1 protein include affinity chromatography techniques, ligand binding assays, etc. Commercial anti-ApoA1 antibodies which can be used in the context of the present invention are available on the market. In a particular embodiment, the levels of protein encoded by the ApoA1 gene are quantified by means of Western blot, immunohistochemical technique, or ELISA.

As it is used herein in the context of the invention, the term "reference value" refers to a laboratory value used as a reference for values/data obtained by means of laboratory examinations of subjects or samples collected from the subjects. The reference value or reference level can be an absolute value, a relative value, a value having an upper and/or lower limit, a range of values, an average value, a median value, an average value, or a value in comparison with a particular control value or baseline value. A reference value can be based on a value of an individual sample, such as, for example, a value obtained for a sample from the subject who is being evaluated, but at a time before developing the disease, or a sample originating from a non-cancerous tissue. The reference value may be based on a large number of samples, for example, a population of subjects of the coinciding chronological age group, or may be based on a group of samples which includes or excludes the sample to be tested. Several considerations are taken into account when determining the reference value. Such considerations include, among others, the age, weight, sex, general physical condition of the patient and the like. For example, a group having the same numbers of at least 2, at least 10, at least 100, and preferably more than 1000 subjects, preferably classified according to the preceding considerations, for example according to various age categories, is taken as a reference group.

The reference value for the ApoA1 level is preferably the mean plasma ApoA 1 level of a group of subjects not suffering from cancer. In another preferred embodiment, the reference value is the mean plasma ApoA 1 level of a group of subjects diagnosed with a primary tumor. In another preferred embodiment, the reference value is the mean plasma ApoA1 level of a group of subjects diagnosed with metastasis.

In another embodiment, the amount of the marker in a sample from a subject can be determined directly in relation to the reference value (for example, in terms of an increase or a decrease, or an increase in the number of folds or a decrease in the number of folds). Advantageously, this may allow comparing the amount of the marker in the sample from the subject with the reference value (i.e., measuring the relative amount of the marker in the sample from the subject with respect to the subject with the reference value) without having to first determine the respective absolute amounts of the marker. Once this reference value has been established, the level of this marker expressed in the tumor tissues of the subjects can be compared with this reference value, and an "increased" or "decreased" level is therefore assigned to it. For example, an increase in expression levels above the reference value of at least 1.1-fold, 1.5-fold, 2-fold, 5-fold, 10-fold, 20-fold, 30-fold, 40-fold, 50-fold, 60-fold, 70-fold, 80-fold, 90-fold, 100-fold, or even higher in comparison with the reference value is considered as an "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold, or even lower in comparison with the reference value is considered as a "decreased" expression level.

### Method for selecting, treatment for a cancer patient (not according to the invention)

The inventors have discovered that patients suffering from metastasis have decreased plasma ApoA 1 levels with respect to patients diagnosed with a primary tumor.

Therefore, a further non-claimed aspect relates to a method for selecting treatment for a cancer patient, where the method comprises determining ApoA1 levels in a biofluid from said patients, wherein decreased ApoA1 levels with respect to a reference value indicate that the therapy of choice is a therapy based on apolipoprotein A, a mimetic peptide thereof, or an activator thereof.

In a disclosure, the cancer is a primary tumor. In another disclosure, the cancer is a metastasis. In a preferred embodiment, the cancer is a colorectal cancer.

The terms "cancer," "colorectal cancer," and "patient" have been described in detail above and are similarly applicable to the methods according to the method of this aspect.

In the context of this aspect, "treatment of cancer" is understood as the administration of apolipoprotein A according to the invention to prevent or delay the onset of symptoms, complications or biochemical indications of the cancer or tumor, to alleviate its symptoms, or to stop or inhibit its development and progression such as the onset of metastasis, for example. The treatment can be a prophylactic treatment for delaying the onset of the disease or for preventing the manifestation of its clinical or subclinical symptoms, or a therapeutic treatment for eliminating or alleviating symptoms after the manifestation of the disease, or in relation to its surgical or radiotherapy treatment. The administration of apolipoprotein A according to the invention can be done simultaneously or consecutively with any other treatment already known in the treatment of cancer.

In the present invention, the term "sample" or "biological sample" refers to biological material isolated from a subject. The biological sample may contain any biological material suitable for detecting the desired marker and may comprise cells and /or non-cellular material of the subject. The sample can be isolated from any suitable biological fluid or tissue, such as blood, blood plasma, serum, urine, cerebrospinal fluid (CSF), or feces, for example. The samples may be biofluid samples. The terms "biological fluid" and "biofluid" are used interchangeably herein and refer to aqueous fluids of biological origin. The biofluid can be obtained from any location (such as blood, plasma, serum, urine, bile, cerebrospinal fluid, vitreous or aqueous humor, or any bodily secretion), an exudate (such as the liquid obtained from an abscess or any other infection or inflammation site), or the liquid obtained from a joint (for example, a normal joint or a joint affected by a disease such as rheumatoid arthritis).

In a preferred embodiment, the biofluid is a plasma sample from the patient.

The ApoA1 protein expression level can be quantified by means of any conventional method which allows detecting and quantifying said protein in a biofluid sample from a subject. In a non-limiting and illustrative manner, the levels of said protein can be quantified, for example, by means of using antibodies capable of binding to ApoA1 (or to fragments thereof containing an antigenic determinant) and the subsequent quantification of the complexes that are formed. The antibodies used in these assays may or may not be labeled. Illustrative examples of markers that can be used include radioactive isotopes, enzymes, fluorophores, chemiluminescent reagents, enzyme substrates or cofactors, enzyme inhibitors, particles, dyes, etc. There is a wide range of known assays that can be used in the present invention, using non-labeled antibodies (primary antibody) and labeled antibodies (secondary antibody); these techniques include, among others, Western blot, ELISA (enzyme-linked immunosorbent assay), RIA (radioimmunoassay), competitive EIA (competitive enzyme immunoassay), DAS-ELISA (double antibody sandwich ELISA), immunocytochemical and immunohistochemical techniques, techniques based on the use of biochips or protein microarrays including specific antibodies, or assays based on colloidal precipitation in formats such as dipsticks. Other ways of detecting and quantifying said ApoA1 protein include affinity chromatography techniques, ligand binding assays, etc. Commercial anti-ApoA1 antibodies which can be used in the context of the present invention are available on the market. In a particular embodiment, the levels of protein encoded by the ApoA1 gene are quantified by means of Western blot, immunohistochemical technique, or ELISA.

As it is used herein in the context of the invention, the term "reference value" refers to a laboratory value used as a reference for values/data obtained by means of laboratory examinations of subjects or samples collected from the subjects. The reference value or reference level can be an absolute value, a relative value, a value having an upper and/or lower limit, a range of values, an average value, a median value, an average value, or a value in comparison with a particular control value or baseline value. A reference value can be based on a value of an individual sample, such as, for example, a value obtained for a sample from the subject who is being evaluated, but at a time before developing the disease, or a sample originating from a non-cancerous tissue. The reference value may be based on a large number of samples, for example, a population of subjects of the coinciding chronological age group, or may be based on a group of samples which includes or excludes the sample to be tested. Several considerations are taken into account when determining the reference value. Such considerations include, among others, the age, weight, sex, general physical condition of the patient and the like. For example, a group having the same numbers of at least 2, at least 10, at least 100, and preferably more than 1000 subjects, preferably classified according to the preceding considerations, for example according to various age categories, is taken as a reference group. The reference value for the ApoA1 level is preferably the mean plasma ApoA 1 level of a group of subjects not suffering from cancer. In another preferred embodiment, the reference value is the mean plasma ApoA 1 level of a group of subjects diagnosed with a primary tumor. In another preferred embodiment, the reference value is the mean plasma ApoA1 level of a group of subjects diagnosed with metastasis.

In another embodiment, the amount of the marker in a sample from a subject can be determined directly in relation to the reference value (for example, in terms of an increase or a decrease, or an increase in the number of folds or a decrease in the number of folds). Advantageously, this may allow comparing the amount of the marker in the sample from the subject with the reference value (i.e., measuring the relative amount of the marker in the sample from the subject with respect to the subject with the reference value) without having to first determine the respective absolute amounts of the marker. Once this reference value has been established, the level of this marker expressed in the tumor tissues of the subjects can be compared with this reference value, and an "increased" or "decreased" level is therefore assigned to it. For example, an increase in expression levels above the reference value of at least 1.1-fold, 1.5-fold, 2-fold, 5-fold, 10-fold, 20-fold, 30-fold, 40-fold, 50-fold, 60-fold, 70-fold, 80-fold, 90-fold, 100-fold, or even higher in comparison with the reference value is considered as an "increased" expression level. On the other hand, a decrease in expression levels below the reference value of at least 0.9-fold, 0.75-fold, 0.2-fold, 0.1-fold, 0.05-fold, 0.025-fold, 0.02-fold, 0.01-fold, 0.005-fold, or even lower in comparison with the reference value is considered as a "decreased" expression level.

In the context of the present invention, apolipoprotein A is selected from ApoA1, ApoA2, ApoA4, and ApoA5. In a particular embodiment, apolipoprotein A is ApoA1.

The terms "apolipoprotein A," "ApoA1," "ApoA2," "ApoA4," and "ApoA5" have been described in detail above and are similarly applicable to the methods according to this aspect.

In the context of the present disclosure, "apolipoprotein A activator" is understood to be any molecule capable of increasing apolipoprotein A messenger RNA expression, as well as increasing apolipoprotein A concentration, giving rise to an increase in the high-density lipoprotein (HDL).

The apolipoprotein A activator of the invention is apabetalone (RVX-208). RVX-208 (2-(4-(2-hydroxyethoxy)-3,5-dimethylphenyl)-5,7-dimethoxyquinazolin-4(3H)-one) is described in detail in Figure 20 of patent document US 8,053,440. Therefore, the apolipoprotein A activator has the following structure:

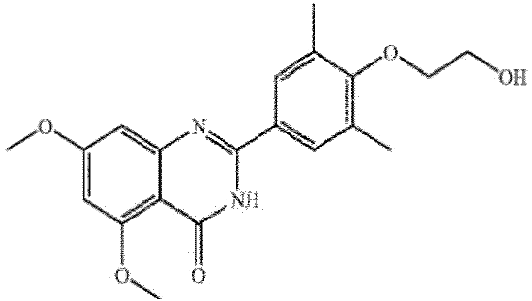

In non-claimed aspect, the term "mimetic peptide" refers to a small chain similar to the protein designed for imitating a peptide. They are typically derived from modifying an existing peptide, or by means of designing similar peptide-imitating systems, such as peptoids and β-peptides. Regardless of the approach, the altered chemical structure is design for advantageously adjusting molecular properties such as biological activity or stability. This may play a role in the development of drug-like compounds from existing peptides. These modifications involve changes in the peptide that will not occur naturally (such as an altered linear structure and the incorporation of non-natural amino acids).

In a preferred non-claimed aspect, the mimetic peptide of ApoA1 is a lipoprotein complex comprising an apolipoprotein ApoA-1 fraction and a lipid fraction.

In the context of the present invention, lipoproteins are macromolecular complexes made up of proteins and lipids. Lipoproteins transport fats throughout the entire body. They are spherical, water-soluble, formed by (a) a non-polar lipid core (esterified cholesterol and triglycerides) and (b) a polar outer layer formed by apoproteins, phospholipids, and free cholesterol. Lipoproteins are classified into different groups according to their density, the higher the density, the higher the protein content (the larger the diameter, the higher the lipid content): chylomicrons, very low-density lipoproteins (VLDLs), intermediate-density lipoproteins (IDLs), low-density lipoproteins (LDLs), and high-density lipoproteins (HDLs). Each type of lipoprotein has a composition and a proportion characteristic of apolipoproteins (ApoA, ApoB, ApoC, ApoE).

The lipid fraction of the lipoprotein complex comprises phosphatidylcholine (lecithin), phosphatidylethanolamine (cephalin), phosphatidylserine, phosphatidylinositol, phosphatidylglycerol, sphingomyelin, ganglioside, cerebroside, or combinations thereof.

The molar ratio of the lipid fraction with respect to the apolipoprotein ApoA-1 fraction may be comprised in the range of about 200:1 to 2:1; about 180:1 to 2:1; about 160:1 to 2:1; about 140:1 to 2:1; about 120:1 to 2:1; about 100:1 to 2:1; about 90:1 to 2:1; about 80:1 to 2:1; about 70:1 to 2:1; about 60:1 to 2:1; about 50:1 to 2:1; about 40:1 to 2:1; about 30:1 to 2:1; about 20:1 to 2:1; about 10:1 to 2:1; about 5:1 to 2:1.

In a preferred embodiment, the lipid fraction of the lipoprotein complex consists essentially of sphingomyelin and about 3% by weight of a negatively charged phospholipid, and the molar ratio of the lipid fraction with respect to the apolipoprotein ApoA-1 fraction is in the range of about 200:1 to 2:1.

In a non-claimed aspect, the mimetic peptide of ApoA 1 is CER-001: (www.cerenis.com/en/our-therapies/cer-001).

CER-001 comprises ApoA1, sphingomyelin and DPPG in a phospholipid: lipoprotein weight ratio of 1:2.7; and with a sphingomyelin:DPPG weight ratio of 97:3. CER-001 is prepared according to the method described in Example 4 of patent document WO 2012/109162.

The invention is described in detail below by means of the following examples which are merely illustrative and in no way seek to limit the scope of the invention.

### EXAMPLES

### Materials and methods

Cell viability and proliferation assays were carried out by means of *XCELLigence* technology (ACEA Biosciences, Inc.) following the manufacturer's indications. Migration and invasion assays were carried out in Coming^{®} BioCoat^{™} Control plates (#354578) and Corning^{®} BioCoat^{™} Matrigel plates (#354480), respectively, following the manufacturer's indications. For the rescue assays, the cells were treated for 72 hours with 30 µM apabetalone (RVX-208; Biovision). Antibodies:ABCA1 (Ab18180, Abcam. 1:500); caveolin-1 (Ab2910, Abcam. 1: 1000); α-tubulin (T9026, Sigma 1: 1000). The DLD1 cells correspond to a human colorectal cancer-derived adherent epithelial cell line (ATCC^{®} CCL-221^{™}). The stable cell lines capable of overexpressing ABCA1 were obtained by means of transduction with genetically modified lentivirus.

### Example 1: ApoA1 protein offers protection against cell malignancy

In relation to the first aspect of this invention, the generation of stable cell lines DLD1 for the overexpression of ApoA1 (Figure 1A) allowed the inventors to observe that even though there are no significant differences in the proliferative capacity (Figure 1B), those cells with increased ApoA1 levels have reduced invasive capacity (Figure 1C).

On the other hand, when analyzing the ApoA1 expression levels in two colorectal cancer patient cohorts from Hospital Universitario de la Paz, the inventors observed how those stage II patients (CRC-TNM Stage II or SII) express, in the tumor tissue, ApoA1 levels that are significantly higher than the corresponding healthy tissue. However, those patients in more advanced, and therefore more invasive, stages have a significant decrease in the ApoA1 messenger RNA levels (Figure 2A). An ELISA analysis of the plasma derived from the patients of the same hospital who had been diagnosed with a primary colon tumor and from the patients having metastasis (Figure 2B) was also carried out. The described results therefore suggest that the ApoA1 protein offers protection against cell malignancy.

### Example 2: ApoA1 protein inhibits DLD1 cell proliferation, migration, and invasion

For the purpose of confirming the preceding results *in vitro,* the inventors carried out cell viability assays which allowed them to demonstrate how the administration of ApoA1 at a concentration of 40 µg/ml in a first dose of 72 hours and at a concentration of 20 µg/ml during result acquisition gives rise to a significant decrease in cell proliferation (Figure 3A). Following the same treatment regimen, the inventors carried out migration and invasion assays. In this case, those cells treated with ApoA1 showed reduced capacities to migrate to the serum-rich region or invade the extracellular matrix (Figure 3B).

### Example 3: RVX-208 inhibits DLD1 cell proliferation

The same assays were repeated by replacing ApoA1 with a treatment with RVX-208. In this case, DLD1 cells were pre-treated for 72 hours before the start of the experiment with RVX-208 at a concentration of 30 µm and were subjected to a treatment with 15 µM of RVX-208 during the development of the experiments. First, as shown in Figure 4A, said treatment caused a significant decrease in proliferative capacity. On the other hand, together with the morphological change observed in the left panels, the treated cells show alterations in the expression levels of genes that are used as markers of the epithelial-mesenchymal transition characteristic of tumor cells; i.e.: an increase in E-cadherin expression levels and a decrease in vimentin levels (Figure 4B). Along with this alteration in these markers, treatment with Rvx-208 significant decreases the migratory and invasive capacity of DLD1 cells (Figure 4C).

### Example 4: ApoA1 protein inhibits the proliferation, migration, and invasion of DLD1 cells expressing ABCA1

When compared with the control situation, DLD1_ABCA1 cells expressing increased levels of the ABCA1 transporter showed greater proliferative capacity (Figure 5A), as well as a significant increase in migration and invasion capacities (Figure 5B). In order to reproduce the best possible physiological situation *in vitro,* these assays were carried out in spheroids derived from the same cell lines embedded in Matrigel (matrix formed by laminin, collagen IV, heparan sulfate, entactin/nidogen, and growth factors). The spheroids derived from cells with high ABCA1 levels show a greater invasive capacity and alterations in the markers of epithelial-mesenchymal transition that has already been discussed in the preceding sections (Figure 5C). As shown in Figure 5D, the cells overexpressing ABCA1 have a cholesterol flux significantly greater than the control DLD 1 cells. When treated with 40 µg/ml of apolipoprotein A1, the proliferative capacity of the DLD1_ABCA1 cells dropped to levels that are comparable to the control situation (Figure 6A). Furthermore, the increase in migratory and invasive capacities was also controlled as a result of the treatment (Figure 6B). Treating the cells with ApoA1 significantly decreases the expression levels of the ABCA1 cholesterol transporter, both on the messenger RNA level and on the protein level (Figure 6C).

### Example 5: RVX-208 inhibits the proliferation ofDLDl cells expressing ABCA1

The inventors were able to confirm said results based on the treatment of DLD1 cells expressing ABCA1 with RVX-208 under the conditions already described in detail in Example 3. In this case, the DLD 1 cells overexpressing ABCA1 were pre-treated for 72 hours before the start of the experiment with RVX-208 at a concentration of 30 µm and were subjected to a treatment with 15 µM of RVX-208 during the development of the experiments. First, as shown in Figure 7A, said treatment caused a significant decrease in proliferative capacity. Furthermore, the increase in migratory and invasive capacities was also controlled as a result of the treatment (Figure 7B). On the other hand, the treated cells show alterations in the expression levels of genes that are used as markers of the epithelial-mesenchymal transition characteristic of tumor cells; i.e.: an increase in E-cadherin expression levels and a decrease in vimentin levels (Figure 7C). The levels of caveolin-1, a protein whose increased levels have been associated with increases in cell migration and invasion, were also analyzed in this experiment. By means of a Western blot assay, the inventors were able to verify that treatment with RVX-208 is capable of reducing the levels of said protein (Figure 7D). This treatment also induces an increase in apolipoprotein A1 expression levels (Figure 7D). Just as direct treatment with ApoA1 induces an increase in ABCA1 levels (Figure 6C), treatment with Rvx-208 reduces ABCA1 expression levels. As a result of this decrease in the expression of the transporter, cholesterol flux decreases significantly both in the control cells and in those overexpressing ABCA1 (Figure 7E). When monitoring the growth of Matrigel-embedded spheroids, the inventors were able to demonstrate that treatment with Rvx-208 slows down the growth of the three-dimensional structures derived from DLD 1 cells overexpressing ABCA1 (Figure 8A).

### Example 6: RVX-208 acts through an increase in ApoA1 levels

For the purpose of confirming that the effects of RVX-208 in cell phenotype recovery were due to the increase in ApoA-1 expression, the inventors inhibited the expression of said protein by means of transfection of a complementary small RNA, ShApoA1 (Figure 9). Twenty-four hours after transfection with ShApoA1 expression vector, the inventors started treatment with 30 µM of RVX-208. Cell viability and proliferation in DLD1 cells (D_NoORF) and D1D1 cell lines expressing increased ApoA1 levels (D_ApoA1) were analyzed with the *xCELLigence* system. The proliferation indices were significantly lower in both cases, i.e., in those cells treated with RVX-208 and in which ApoA1 expression (Scr + Rvx-208) had not been inhibited. Furthermore, the interference of ApoA1 expression with shApoA1 causes a significant increase in the proliferation levels of the control cells. Similarly, this silencing of the control cells and of cells with high ABCA1 levels reduces the effectiveness of treatment with Rvx-208 in terms of a decrease in cell invasion capacities. Both results demonstrate that the antiproliferative and invasion inhibition effects of RVX-208 are due to an increase in apolipoprotein A1 expression levels.

## Claims

1. A composition comprising an apolipoprotein A activator with the following structure: 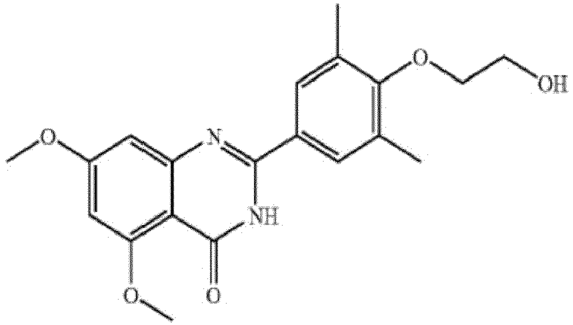 for use in a method of inhibiting the proliferation, migration, and invasion of cancer cells, in a cancer patient with decreased ApoA1 plasma levels with respect to a reference value, wherein reference value for the ApoA1 level is the mean plasma ApoA1 level of a group of subjects not suffering from cancer, and wherein said cancer cells are **characterized by** having an overexpression of ABCA1.

2. The composition for use according to claim 1, wherein the cancer cells are colorectal cancer cells.

3. The composition for use according to any of claims 1 to 2, wherein the apolipoprotein A activator forms a lipoprotein complex comprising a fraction comprising said apolipoprotein A activator and a lipid fraction.

4. The composition for use according to claim 3, wherein the lipid fraction of the lipoprotein complex consists essentially of sphingomyelin and about 3% by weight of a negatively charged phospholipid, and the molar ratio of the lipid fraction with respect to the apolipoprotein A activator fraction is in the range of about 200:1 to 2:1.

## Patentansprüche

1. Zusammensetzung umfassend einen Apolipoprotein A-Aktivator mit der folgenden Struktur: 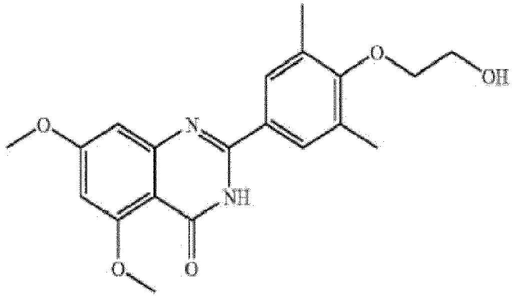 für deren Verwendung in einem Verfahren zur Hemmung der Proliferation, Migration und Invasion von Krebszellen, in einem Krebspatienten mit verringerten ApoAl-Plasmaspiegeln in Bezug auf einen Referenzwert, wobei der Referenzwert für den ApoA1-Spiegel der durchschnittliche ApoAl-Plasmaspiegel einer Gruppe von Individuen ist, welche nicht unter Krebs leiden, und wobei die genannten Krebszellen **dadurch gekennzeichnet sind, dass** sie eine Überexpression von ABCA1 aufweisen.

2. Zusammensetzung für deren Verwendung nach Anspruch 1, wobei die Krebszellen Kolorektalkrebszellen sind.

3. Zusammensetzung für deren Verwendung nach einem der Ansprüche 1 bis 2, wobei der Apolipoprotein A-Aktivator einen Lipoproteinkomplex bildet, umfassend eine Fraktion, welche den genannten Apolipoprotein A-Aktivator und eine Lipidfraktion umfasst.

4. Zusammensetzung für deren Verwendung nach Anspruch 3, wobei die Lipidfraktion des Lipoproteinkomplexes im Wesentlichen aus Sphingomyelin und etwa 3 Gew.-% eines negativ geladenen Phospholipids besteht, und das Molarverhältnis der Lipidfraktion in Bezug auf die Apolipoprotein A-Aktivator-Fraktion im Bereich von etwa 200:1 bis 2:1 liegt.

## Revendications

1. Composition comprenant un activateur d'apolipoprotéine A avec la structure suivante : 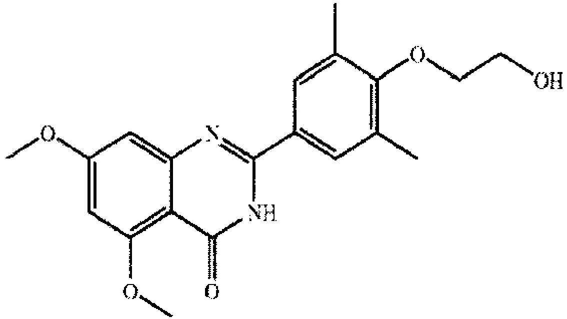 pour son utilisation dans une méthode d'inhibition de la prolifération, de la migration et de l'invasion des cellules cancéreuses, chez un patient cancéreux avec des niveaux plasmatiques d'Apo A1 diminués par rapport à une valeur de référence, dans laquelle la valeur de référence pour le niveau d'Apo A1 est le niveau plasmatique moyen d'Apo A1 d'un groupe de sujets ne souffrant pas de cancer, et dans laquelle lesdites cellules cancéreuses sont **caractérisées par** avoir une surexpression d'ABCA1.

2. Composition pour son utilisation selon la revendication 1, dans laquelle les cellules cancéreuses sont des cellules cancéreuses colorectales.

3. Composition pour son utilisation selon l'une quelconque des revendications 1 à 2, dans laquelle l'activateur d'apolipoprotéine A forme un complexe lipoprotéique comprenant une fraction comprenant ledit activateur d'apolipoprotéine A et une fraction lipidique.

4. Composition selon la revendication 3, dans laquelle la fraction lipidique du complexe lipoprotéique consiste essentiellement en sphingomyéline et en environ 3 % en poids d'un phospholipide chargé négativement, et le rapport molaire de la fraction lipidique par rapport à la fraction d'activateur d'apolipoprotéine A est dans la plage d'environ 200:1 à 2:1.
